# EUROPEAN PATENT APPLICATION

(11) **EP 3 053 510 A1**
(43) Date of publication of application: **10.08.2016**
(21) Application number: 14851386.4
(22) Date of filing: 02.09.2014
(51) Int. Cl.: A61B 1/04, A61B 1/00, H04N 5/225, H04N 5/232, H04N 7/18

(54) **DATA RECEPTION DEVICE, CAPSULE ENDOSCOPE SYSTEM, DATA RECEPTION METHOD, AND PROGRAM**

(30) Priority: 02.10.2013 JP 2013207528
(71) Applicant: Olympus Corporation, Hachioji-shi Tokyo 192-8507 (JP)
(72) Inventor: YANAGIDATE, Masaharu, Tokyo 192-8507 (JP)
(74) Representative: Gunzelmann, Rainer
(86) International application number: PCT/JP2014/073003
(87) International publication number: WO 2015/049947

(57) **Abstract**

A data reception device includes a wireless communication interface configured to receive imaging data transmitted from a capsule endoscope in which a frame rate of imaging is changeable based on a frame rate-setting value for designating the frame rate and which transmits the imaging data after the imaging by performing the imaging, and to transmit the frame rate-setting value to the capsule endoscope, a storage module configured to store reference image information to be used to specify a part of a living body included in the imaging data, a range-of-interest detection unit configured to detect a range of interest which is a range of the part in the imaging data based on the reference image information, an amount-of-change detection unit configured to detect an amount of change in the range of interest between the imaging data of two different frames, and a frame rate determination unit configured to determine the frame rate-setting value to be transmitted to the capsule endoscope as a value greater than a previously determined frame rate-setting value when the amount of the change in the range of interest exceeds a predetermined reference value, and to determine the frame rate-setting value to be transmitted to the capsule endoscope as a value less than the previously determined frame rate-setting value when the amount of the change in the range of interest is less than the predetermined reference value.

## Description

### [Technical Field]

The present invention relates to technology for receiving imaging data transmitted from a capsule endoscope and controlling a frame rate of the capsule endoscope.

Priority is claimed on Japanese Patent Application No. 2013-207528, filed October 2, 2013, the content of which is incorporated herein by reference.

### [Background Art]

In Patent Literature 1, an example in which an external receiver receiving a captured image transmitted from an in vivo imaging device detects a defect or a color of a structure of an organ such as an intestine and changes an operation mode of the imaging device is disclosed. Also, in Patent Literature 1, a process of increasing an imaging speed (frame acquisition speed) of an image sensor is disclosed as an example in which the operation mode changes.

### [Citation List]

### [Patent Literature]

### [Patent Literature 1]

Published Japanese Translation No. 2006-509574 of the PCT International Publication

### [Summary of Invention]

### [Technical Problem]

When the conventional technology is used, it is possible to increase the imaging speed of the image sensor during imaging of a lesion part when the lesion part is imaged. However, power is consumed in capturing and transmission of the same image because a high imaging speed is maintained even when there is no change in the lesion part during the imaging.

The present invention has been made in view of the above-described problem, and an objective thereof is to reduce power consumption while maintaining a high imaging speed according to necessity.

### [Solution to Problem]

According to a first aspect of the present invention, a data reception device includes: a wireless communication interface configured to receive imaging data transmitted from a capsule endoscope in which a frame rate of imaging is changeable based on a frame rate-setting value for designating the frame rate and which transmits the imaging data after the imaging by performing the imaging, and to transmit the frame rate-setting value to the capsule endoscope; a storage module configured to store reference image information to be used to specify a part of a living body included in the imaging data; a range-of-interest detection unit configured to detect a range of interest which is a range of the part in the imaging data based on the reference image information; an amount-of-change detection unit configured to detect an amount of change in the range of interest between the imaging data of two different frames; and a frame rate determination unit configured to determine the frame rate-setting value to be transmitted to the capsule endoscope as a value greater than a previously determined frame rate-setting value when the amount of the change in the range of interest exceeds a predetermined reference value, and to determine the frame rate-setting value to be transmitted to the capsule endoscope as a value less than the previously determined frame rate-setting value when the amount of the change in the range of interest is less than the predetermined reference value.

According to a second aspect of the present invention, in the data reception device according to the first aspect, the frame rate determination unit may determine the frame rate-setting value to be transmitted to the capsule endoscope as a predetermined value when no range of interest is detected, set a lower limit value greater than the predetermined value when the range of interest is detected, and determine the frame rate-setting value to be transmitted to the capsule endoscope as a value which corresponds to the amount of the change in the range of interest and being greater than or equal to the lower limit value.

According to a third aspect of the present invention, in the data reception device according to the first aspect, the range-of-interest detection unit may further determine whether the range of interest is located in a first region in which a relatively distant view is imaged or a second region in which a relatively close view is imaged when the range of interest is detected. When it is determined that the range of interest is located in the second region, the frame rate determination unit may further determine the frame rate-setting value to be transmitted to the capsule endoscope as a value which corresponds to the amount of the change in the range of interest and being greater than the frame rate-setting value determined when it is determined that the range of interest is located in the first region.

According to a fourth aspect of the present invention, in the data reception device according to the third aspect, the first region may be a region which is a part of an image based on the imaging data and includes a center of the image.

According to a fifth aspect of the present invention, in the data reception device according to the third aspect, when the range of interest is first detected after a point in time at which the detection of the range of interest starts, the frame rate determination unit may determine the frame rate-setting value to be transmitted to the capsule endoscope as a first predetermined value when it is determined that the detected range of interest is located in the first region, and may determine the frame rate-setting value to be transmitted to the capsule endoscope as a second predetermined value greater than the first predetermined value when it is determined that the detected range of interest is located in the second region.

According to a sixth aspect of the present invention, the data reception device according to the first aspect may further include: an amount-of-background-change detection unit configured to detect an amount of change in a background except the range of interest between the imaging data of the two different frames. The frame rate determination unit may determine the frame rate-setting value to be transmitted to the capsule endoscope as a value corresponding to the amount of the change in the range of interest and the amount of the change in the background.

According to a seventh aspect of the present invention, in the data reception device according to the sixth aspect, the frame rate determination unit may determine the frame rate-setting value to be transmitted to the capsule endoscope corresponding to the amount of the change in the range of interest as a value reflecting the amount of the change in the background at a first degree of influence when the range of interest is detected, and may determine the frame rate-setting value to be transmitted to the capsule endoscope as a value reflecting the amount of the change in the background at a second degree of influence less than the first degree of the influence when no range of interest is detected.

According to an eighth aspect of the present invention, in the data reception device according to the first aspect, the storage module may store a plurality of pieces of the reference image information. The reference value may be set to be changeable for each piece of the reference image information.

According to a ninth aspect of the present invention, a capsule endoscope system including a capsule endoscope and a data reception device, wherein the capsule endoscope includes: an imaging module in which a frame rate of imaging is changeable based on a frame rate-setting value for designating the frame rate and configured to output imaging data after the imaging by performing the imaging; and a first wireless communication interface configured to transmit the imaging data output from the imaging module to the data reception device and receive the frame rate-setting value from the data reception device, and wherein the data reception device includes: a second wireless communication interface configured to receive the imaging data from the capsule endoscope and transmit the frame rate-setting value to the capsule endoscope; a storage module configured to store reference image information to be used to specify a part of a living body among the imaging data; a range-of-interest detection unit configured to detect a range of interest which is a range of the part in the imaging data based on the reference image information; an amount-of-change detection unit configured to detect an amount of change in the range of interest between the imaging data of two different frames; and a frame rate determination unit configured to determine the frame rate-setting value to be transmitted to the capsule endoscope as a value greater than a previously determined frame rate-setting value when the amount of the change in the range of interest exceeds a predetermined reference value and determine the frame rate-setting value to be transmitted to the capsule endoscope as a value less than the previously determined frame rate-setting value when the amount of the change in the range of interest is less than the predetermined reference value.

According to a tenth aspect of the present invention, a data reception method is provided including the steps of: receiving, by a wireless communication interface, imaging data transmitted from a capsule endoscope in which a frame rate of imaging is changeable based on a frame rate-setting value for designating the frame rate and which transmits imaging data after the imaging by performing the imaging; detecting, by a range-of-interest detection unit, a range of interest which is a range of a part of a living body in the imaging data based on reference image information of a storage module configured to store the reference image information to be used to specify the part among the imaging data; detecting, by an amount-of-change detection unit, an amount of change in the range of interest between the imaging data of two different frames; determining, by a frame rate determination unit, the frame rate-setting value to be transmitted to the capsule endoscope as a value greater than a previously determined frame rate-setting value when the amount of the change in the range of interest exceeds a predetermined reference value and determining the frame rate-setting value to be transmitted to the capsule endoscope as a value less than the previously determined frame rate-setting value when the amount of the change in the range of interest is less than the predetermined reference value; and transmitting, by the wireless communication interface, the determined frame rate-setting value to the capsule endoscope.

According to an eleventh aspect of the present invention, a program is provided for causing a computer to execute the steps of: causing a wireless communication interface to receive imaging data transmitted from a capsule endoscope in which a frame rate of imaging is changeable based on a frame rate-setting value for designating the frame rate and which transmits imaging data after the imaging by performing the imaging; detecting a range of interest which is a range of a part of a living body in the imaging data based on reference image information of a storage module configured to store the reference image information to be used to specify the part among the imaging data; detecting an amount of change in the range of interest between the imaging data of two different frames; determining the frame rate-setting value to be transmitted to the capsule endoscope as a value greater than a previously determined frame rate-setting value when the amount of the change in the range of interest exceeds a predetermined reference value and determining the frame rate-setting value to be transmitted to the capsule endoscope as a value less than the previously determined frame rate-setting value when the amount of the change in the range of interest is less than the predetermined reference value; and causing the wireless communication interface to transmit the determined frame rate-setting value to the capsule endoscope.

### [Advantageous Effects of Invention]

According to the above-described data reception device, capsule endoscope system, data reception method, and program, it is possible to reduce power consumption while maintaining a high imaging speed according to necessity by determining a frame rate-setting value to be transmitted to a capsule endoscope as a value greater than a previously determined frame rate-setting value when an amount of change in a range of interest exceeds a predetermined reference value and determining the frame rate-setting value to be transmitted to the capsule endoscope as a value less than the previously determined frame rate-setting value when the amount of the change in the range of interest is less than the predetermined reference value.

### [Brief Description of Drawings]

Fig. 1 is a block diagram illustrating a configuration of a capsule endoscope system according to a first embodiment of the present invention.
Fig. 2 is a block diagram illustrating a configuration of a capsule endoscope according to the first embodiment of the present invention.
Fig. 3 is a block diagram illustrating a configuration of a reception device according to the first embodiment of the present invention.
Fig. 4 is a reference diagram illustrating a range of imaging data and movement of a range of interest in a captured image in the first embodiment of the present invention.
Fig. 5 is a reference diagram illustrating a change in an area of the range of interest in the first embodiment of the present invention.
Fig. 6 is a graph illustrating the change in the area of the range of interest in the first embodiment of the present invention.
Fig. 7 is a graph illustrating a change in a frame rate in the first embodiment of the present invention.
Fig. 8 is a reference diagram illustrating a range of imaging data and movement of a range of interest in a captured image in the first embodiment of the present invention.
Fig. 9 is a reference diagram illustrating a change in an area of the range of interest in the first embodiment of the present invention.
Fig. 10 is a graph illustrating the change in the area of the range of interest in the first embodiment of the present invention.
Fig. 11 is a graph illustrating a change in a frame rate in the first embodiment of the present invention.
Fig. 12 is a flowchart illustrating a procedure of a frame rate-setting process in the first embodiment of the present invention.
Fig. 13 is a graph illustrating a change in an area of a range of interest in a second embodiment of the present invention.
Fig. 14 is a graph illustrating a change in a frame rate in the second embodiment of the present invention.
Fig. 15 is a flowchart illustrating a procedure of a frame rate-setting process in the second embodiment of the present invention.
Fig. 16 is a reference diagram illustrating a range of imaging data in a third embodiment of the present invention.
Fig. 17 is a reference diagram illustrating the range of the imaging data and movement of a range of interest in a captured image in the third embodiment of the present invention.
Fig. 18 is a graph illustrating a change in an area of the range of interest in the third embodiment of the present invention.
Fig. 19 is a graph illustrating a change in a frame rate in the third embodiment of the present invention.
Fig. 20 is a reference diagram illustrating the range of the imaging data and movement of the range of interest in a captured image in the third embodiment of the present invention.
Fig. 21 is a graph illustrating a change in an area of a range of interest in the third embodiment of the present invention.
Fig. 22 is a graph illustrating a change in a frame rate in the third embodiment of the present invention.
Fig. 23 is a flowchart illustrating a procedure of a frame rate-setting process in the third embodiment of the present invention.
Fig. 24 is a block diagram illustrating a configuration of a capsule endoscope according to a fourth embodiment of the present invention.
Fig. 25 is a flowchart illustrating a procedure of a frame rate-setting process in the fourth embodiment of the present invention.

### [Description of Embodiments]

Hereinafter, embodiments of the present invention will be described with reference to the drawings.

### (First embodiment)

The first embodiment of the present invention will be described. In this embodiment, an example in which the present invention is applied to a capsule endoscope system having a capsule endoscope and a reception device will be described. In the capsule endoscope, a frame rate of imaging is changeable by a wireless instruction from the reception device. The capsule endoscope transmits imaging data which is image data after the imaging to the reception device using wireless communication. The reception device receives the imaging data transmitted from the capsule endoscope and controls a frame rate of the capsule endoscope.

### [Configuration]

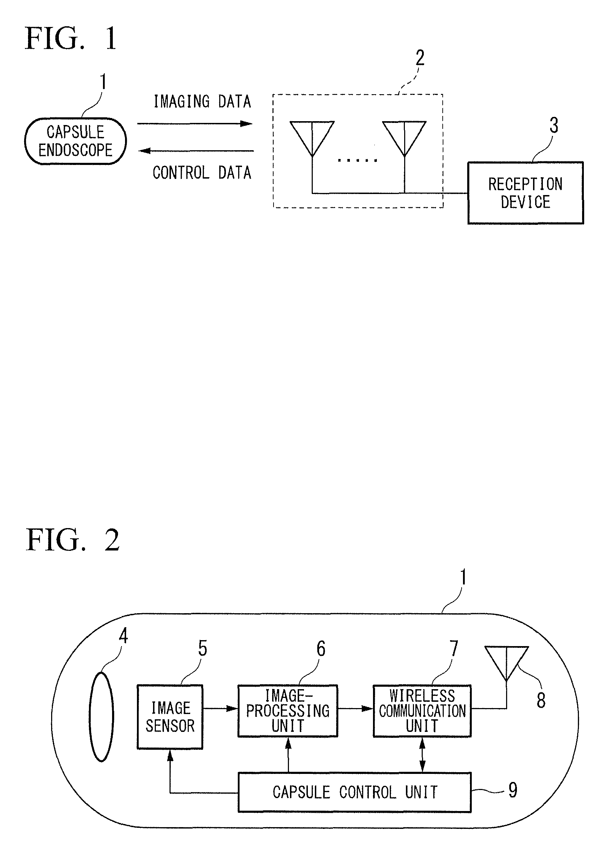

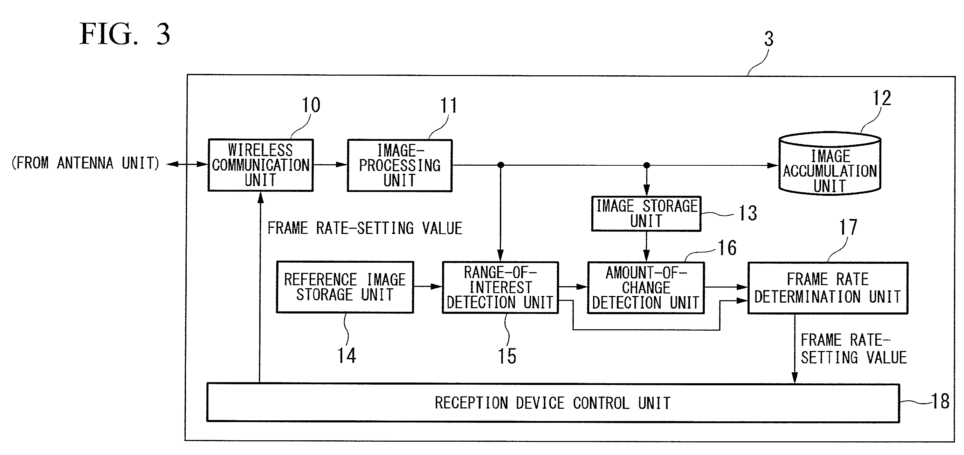

Fig. 1 illustrates a configuration of a capsule endoscope system according to this embodiment. Fig. 2 illustrates a configuration of a capsule endoscope 1 according to this embodiment. Fig. 3 illustrates a configuration of a reception device 3 according to this embodiment. First, the configurations of the capsule endoscope system, the capsule endoscope 1, and the reception device 3 and outlines of operations of the capsule endoscope 1 and the reception device 3 will be described using Figs. 1 to 3.

The capsule endoscope system according to this embodiment has the capsule endoscope 1, the reception device 3, and an antenna unit 2 having a plurality of antennas as illustrated in Fig. 1. The antenna unit 2 is connected to the reception device 3, and the capsule endoscope 1 and the reception device 3 are connected by wireless communication performed via the antenna unit 2. Although the antenna unit 2 and the reception device 3 are separated and the reception device 3 itself does not include any antenna in this example, the reception device 3 itself may include an antenna.

The capsule endoscope 1 is inserted into a living body (human body) and wirelessly transmits a captured image. The wirelessly transmitted captured image is received through the antenna unit 2. A process in which the reception device 3 selects and receives a signal from an antenna having highest reception sensitivity is performed. The reception device 3 stores the received captured image and detects a lesion part or an examination target part of the captured image. Further, the reception device 3 determines a frame rate at which an amount of change between captured images of two different frames becomes a desired value and sets the determined frame rate in the capsule endoscope 1. Because the configuration of the entire capsule endoscope system is well known, further description thereof will be omitted.

As illustrated in Fig. 2, the capsule endoscope 1 includes a lens 4, an image sensor 5, an image-processing unit 6, a wireless communication unit 7, an antenna unit 8, and a capsule control unit 9. The lens 4 forms an image of light from the lesion part or the examination target part on the image sensor 5. The image sensor 5 is an imaging module in which a frame rate of imaging is changeable based on a frame rate-setting value for designating the frame rate and configured to output imaging data after imaging by performing the imaging. An image within a body is captured by the lens 4 and the image sensor 5 and imaging data is transmitted to the image-processing unit 6.

The image-processing unit 6 performs image processing (compression processing) on imaging data output from the image sensor 5. After the compression processing is performed by the image-processing unit 6, data after the compression processing is sent to the wireless communication unit 7. The wireless communication unit 7 wirelessly communicates with the reception device 3 via the antenna unit 8. The wireless communication unit 7 and the antenna unit 8 are wireless communication interfaces configured to transmit the imaging data output from the imaging module (data processed by the image-processing unit 6) to the reception device 3 and receive a frame rate-setting value from the reception device 3. Packetizing and high-frequency processing are performed in the wireless communication unit 7 and a radio signal is transmitted via the antenna unit 8. The wireless communication unit 7 receives the frame rate-setting value transmitted from the reception device 3 via the antenna unit 8 and notifies the capsule control unit 9 of the received frame rate-setting value.

The capsule control unit 9 controls each part within the capsule endoscope 1. The capsule control unit 9 sets the frame rate when the image sensor 5 performs imaging based on the frame rate-setting value of the notification from the wireless communication unit 7. The capsule control unit 9 controls the operation of the image sensor 5 so that the imaging is performed at the set frame rate. A power source of each part is supplied by a battery (not illustrated). Because the capacity of the battery is limited, it is necessary to perform imaging at as low a frame rate as possible when capturing an unimportant image or an image without change.

As illustrated in Fig. 3, the reception device 3 includes a wireless communication unit 10, an image-processing unit 11, an image accumulation unit 12, an image storage unit 13, a reference image storage unit 14, a range-of-interest detection unit 15, an amount-of-change detection unit 16, a frame rate determination unit 17, and a reception device control unit 18. The wireless communication unit 10 wirelessly communicates with the capsule endoscope 1 via the antenna unit 2. The wireless communication unit 10 is a wireless communication interface configured to receive imaging data transmitted from the capsule endoscope 1 and transmit a frame rate-setting value to the capsule endoscope 1.

The image-processing unit 11 performs image processing (decompression processing) on the imaging data received by the wireless communication unit 10. The imaging data processed by the image-processing unit 11 is output to the image accumulation unit 12, the image storage unit 13, and the range-of-interest detection unit 15. The image accumulation unit 12 is a storage module configured to generate and accumulate a file of all imaging data output from the capsule endoscope 1 in one examination. The image storage unit 13 is a storage module configured to temporarily store the imaging data for use in the frame rate setting to be described below. The reference image storage unit 14 is a storage module configured to store reference image information (for example, feature information of an image of a lesion part or an examination target part) to be used to specify a part (the lesion part or the examination target part) of a living body among imaging data. Two or more of the image accumulation unit 12, the image storage unit 13, and the reference image storage unit 14 may be constituted of one storage module.

The range-of-interest detection unit 15 detects a range of interest which is a range of the lesion part or the examination target part in the imaging data based on the reference image information stored in the reference image storage unit 14. More specifically, the range-of-interest detection unit 15 stores imaging data for one frame output from the image-processing unit 11 and compares the stored imaging data with the reference image information stored in the reference image storage unit 14, thereby specifying the presence/absence and position of the range of interest (the position within the captured image). The range-of-interest detection unit 15 outputs information indicating the presence/absence of the range of interest to the amount-of-change detection unit 16 and the frame rate determination unit 17. The range-of-interest detection unit 15 outputs information about the specified position as information about the range of interest to the amount-of-change detection unit 16 when the range of interest is present.

A technique using the principle of pattern matching or the like is proposed as an algorithm for detecting the range of interest. The detecting algorithm itself is not a feature of the present invention and a well-known technique is used in the detecting algorithm. Further description of the detecting algorithm will be omitted.

Imaging data of a current frame and imaging data of a previous frame are input from the image storage unit 13 to the amount-of-change detection unit 16. The amount-of-change detection unit 16 detects an amount of change in the range of interest between imaging data of two different frames. More specifically, the amount-of-change detection unit 16 detects the amount of the change by comparing an image within the range of interest of the notification from the range-of-interest detection unit 15 between frames and outputs the detected amount of the change to the frame rate determination unit 17. For example, a change in an area of the range of interest, a change in a color tone, a change in luminance (illumination state), or the like is considered as a change in the image. According to examination content, any one of the changes is selected.

In this embodiment, description focusing on only the "change in the area of the range of interest" will be given. In the following description, the amount of the change detected by the amount-of-change detection unit 16 is an amount of the change in the area of the range of interest.

The frame rate determination unit 17 determines a frame rate-setting value at which the amount of the change detected by the amount-of-change detection unit 16 becomes a predetermined value and outputs the determined frame rate-setting value to the reception device control unit 18. More specifically, when the amount of the change in the range of interest exceeds a predetermined reference value, the frame rate determination unit 17 determines the frame rate-setting value to be transmitted to the capsule endoscope 1 as a value greater than a previously determined frame rate-setting value (for example, a current frame rate-setting value). When the amount of the change in the range of interest is less than the reference value, the frame rate determination unit 17 determines the frame rate-setting value to be transmitted to the capsule endoscope 1 as a value less than the previously determined frame rate-setting value. For example, the frame rate determination unit 17 determines a first frame rate-setting value. Thereafter, when the amount of the change in the range of interest exceeds the predetermined reference value, the frame rate determination unit 17 determines a second frame rate-setting value to be transmitted to the capsule endoscope 1 as a value greater than the first frame rate-setting value. When the amount of the change in the range of interest is less than the reference value, the frame rate determination unit 17 determines the second frame rate-setting value to be transmitted to the capsule endoscope 1 as a value less than the first frame rate-setting value. The frame rate determination unit 17 internally holds the determined frame rate-setting value. The reception device control unit 18 is notified of the determined frame rate-setting value.

The reception device control unit 18 controls each part within the reception device 3. The reception device control unit 18 outputs the frame rate-setting value of the notification from the frame rate determination unit 17 and controls the wireless communication unit 10. The wireless communication unit 10 transmits the frame rate-setting value to the capsule endoscope 1 via the antenna unit 2. The frame rate-setting value transmitted to the capsule endoscope 1 is used as the frame rate value in the next imaging.

The reception device control unit 18 stores a program for controlling the operation of the reception device control unit 18 or necessary data. For example, the reception device control unit 18 which is a computer of the reception device 3 reads and executes the program for controlling the operation of the reception device control unit 18, so that functions of the range-of-interest detection unit 15, the amount-of-change detection unit 16, and the frame rate determination unit 17, for example, can be implemented as a function of software. This program, for example, may be provided by a "computer-readable recording medium" such as a flash memory. The above-described program may be input to the reception device 3 by transmitting the program from the computer storing the program in a storage device or the like via a transmission medium or transmitting the program to the reception device 3 by transmission waves in a transmission medium. Here, the "transmission medium" for transmitting the program refers to a medium having a function of transmitting information, such as a network (communication network) like the Internet or a communication circuit (communication line) like a telephone circuit. In addition, the above-described program may be a program for implementing some of the above-described functions. Further, the above-described program may be a program, i.e., a so-called differential file (differential program), capable of implementing the above-described function in combination with a program already recorded on the computer.

A device including the wireless communication unit 10, the reference image storage unit 14, the range-of-interest detection unit 15, the amount-of-change detection unit 16, and the frame rate determination unit 17 as a minimum configuration corresponds to an aspect of a data reception device of the present invention. For example, the wireless communication unit 10 corresponds to a wireless communication interface in the data reception device of the present invention, the reference image storage unit 14 corresponds to the storage module in the data reception device of the present invention, the range-of-interest detection unit 15 corresponds to the range-of-interest detection unit in the data reception device of the present invention, the amount-of-change detection unit 16 corresponds to the amount of change detection unit in the data reception device of the present invention, and the frame rate determination unit 17 corresponds to the frame rate determination unit in the data reception device of the present invention.

A capsule endoscope system having a device including the wireless communication unit 10, the reference image storage unit 14, the range-of-interest detection unit 15, the amount-of-change detection unit 16, and the frame rate determination unit 17 as the minimum configuration and a capsule endoscope including the image sensor 5, the wireless communication unit 7, and the antenna unit 8 as the minimum configuration corresponds to an aspect of the capsule endoscope system according to the present invention. A configuration corresponding to the reception device provided in the capsule endoscope system according to the present invention is as described above. For example, the image sensor 5 corresponds to an imaging module in the capsule endoscope provided in the capsule endoscope system according to the present invention. The wireless communication unit 7 and the antenna unit 8 correspond to a first wireless communication interface in the capsule endoscope provided in the capsule endoscope system according to the present invention.

Through the above-described configuration, the frame rate-setting value is set to a value greater than a current setting value when the amount of the change in the range of interest exceeds the reference value and the change in the image between frames increases. Thus, the capsule endoscope system according to the present invention can maintain a high imaging speed. When the amount of the change in the range of interest is less than the reference value and the change in the image between the frames decreases, the frame rate-setting value is set to a value less than the current setting value. Thus, the capsule endoscope system according to the present invention can reduce power consumption.

### [Frame rate-setting operation]

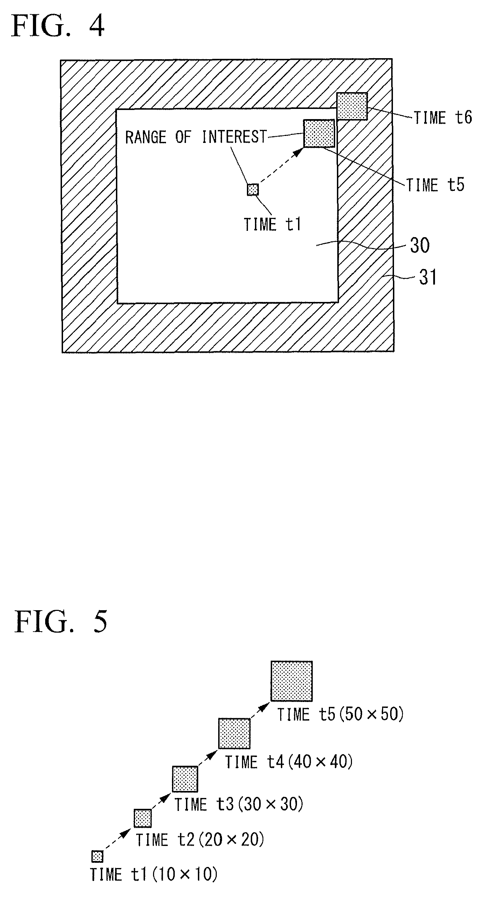

A method of setting a frame rate in this embodiment will be described using Figs. 4 to 7. In this description, description of some of operations of the device will be simplified to easily convey the spirit or scope of the present invention.

Fig. 4 illustrates a range of imaging data to be transmitted from the capsule endoscope 1 to the reception device 3 and movement of a range of interest in a captured image. In this example, the imaging data is data of a rectangular effective imaging region 30 (480x480 pixels) at the center of the captured image illustrated in Fig. 4. In Fig. 4, a region 31 outside the effective imaging region 30 is a region outside an imaging range. Imaging data of the region 31 is not transmitted to the reception device 3.

An example of movement of the range of interest in accordance with the passage of time is illustrated in Fig. 4. If the lens 4 or the like is directed in a traveling direction when the capsule endoscope 1 passes through an intestinal tract of a small intestine or the like, a state in which the range of interest moves from the center to the periphery along with the passage of time is imaged as illustrated in Fig. 4. In this case, the capsule endoscope 1 moves forward (in an imaging direction of the image sensor 5).

The movement of the range of interest when the capsule endoscope 1 moves at a uniform speed is illustrated in Fig. 4. Initially, the range of interest is detected in the vicinity of the center of a captured image at time t1. The capsule endoscope 1 approaches a part of the range of interest along with the passage of time and the range of interest in the captured image moves to a peripheral part. The range of interest is positioned at the end of the effective imaging region 30 at time t5 and is outside the effective imaging region 30 at time t6.

Fig. 5 illustrates a detailed example of a change in an area of the range of interest which moves as illustrated in Fig. 4. In this description, description will be given under the assumption that an interval of each of times t1 to t6 is 1 sec. The area (the number of pixels) of the range of interest is enlarged as the capsule endoscope 1 approaches the part of the range of interest. As illustrated in Fig. 5, the area of the range of interest sequentially changes to 10×10 (t1) → 20×20 (t2) → 30×30 (t3) → 40×40 (t4) → 50×50 (t5). In order to simplify the description, the description will be given under the assumption that the area of the range of interest linearly changes between times.

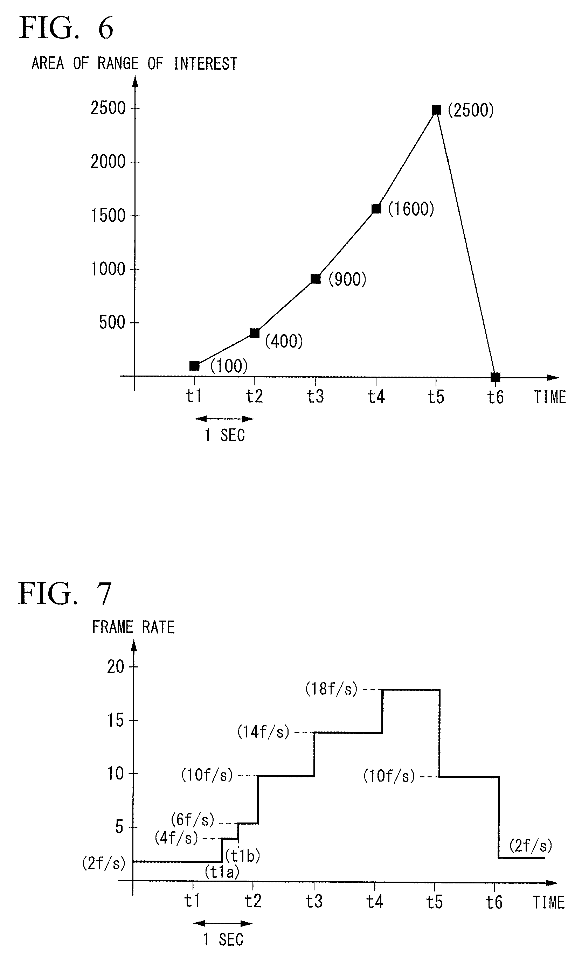

Fig. 6 illustrates an area of the range of interest at each time and a change in the area between times when the area of the range of interest changes as illustrated in Fig. 5. The horizontal axis of Fig. 6 represents time and the vertical axis of Fig. 6 represents an area of a range of interest. A change in the area between the times is linearly approximated.

Hereinafter, an example in which a value of 50 is set as a reference value so that an amount of change in a range of interest between frames becomes 50 pixels will be described. Although the reference value is 50 as an example in this description, the reference value is variable and determined according to a type of reference image information. More specifically, the reference image storage unit 14 stores a plurality of pieces of the reference image information and the reference value is set to be changeable for each piece of the reference image information. For example, the reference value is stored in the reference image storage unit 14 in association with the reference image information. The reference value corresponding to the used reference image information is output to the frame rate determination unit 17. Alternatively, the reference value is stored in the reception device control unit 18 along with information in which the reference value and the reference image information are associated. The reference value corresponding to the used reference image information is output to the frame rate determination unit 17.

For example, a small value is used as a reference value corresponding to reference image information of a symptom for which a lesion part is relatively small. In this case, even when the amount of the change in the range of interest is small, the frame rate is set to a large value and imaging is performed at a high frame rate.

In this case, a medical worker who is a user can perform the selection of the reference image information or the change in the reference value for the selected reference image information according to a symptom of a patient before the initiation of photography by the capsule endoscope. An upper limit of the frame rate settable in the capsule endoscope 1 according to this embodiment is 30 frames/second (f/s). When the frame rate-setting value exceeds the upper limit of 30 f/s in the computation of the frame rate-setting value, the frame rate-setting value is set to 30 f/s. The upper limit of the frame rate of 30 f/s is an example and the upper limit of the frame rate may be a value other than the value of 30 f/s.

Fig. 7 illustrates an example of the frame rate corresponding to the change in the area of the range of interest illustrated in Fig. 6. The horizontal axis of Fig. 7 represents time and the vertical axis of Fig. 7 represents a frame rate (f/s). Before the range of interest is detected, the frame rate is fixed at 2 f/s. In a frame before time t1, the area of the range of interest is 0 (pixels). Because the range of interest is first detected and the area of the range of interest is 100 (pixels) at time t1, the amount of the change in the range of interest between frames is 100.

When the next set frame rate is denoted by x, x is computed as follows. Because the amount of the change in the range of interest between the frames is 100 when imaging is performed at a frame rate of 2 f/s, x = 2 (f/s)× 100/50 = 4 to set the amount of the change in the range of interest between the frames to 50 (hereinafter, Formula A). Consequently, 4 f/s is set as the next frame rate.

The frame rate is set immediately after the computation of the frame rate. Because the computation of the frame rate is performed immediately after time t1 in the above-described case, the frame rate changes to 4 f/s from a frame for which imaging starts immediately after a period of one frame has elapsed from time t1. In this case, because the frame rate is 2 f/s at time t1, the frame rate changes to 4 f/s at time t1a at which 0.5 sec, which is the period of the one frame, has elapsed from time t1.

As illustrated in Fig. 7, imaging continues at the frame rate of 2 f/s immediately after time t1. Because the area of the range of interest linearly changes at a speed of 300/s during a period of times t1 to t2 (1 sec), the amount of the change in the range of interest between frames is 300/2 = 150 when the frame rate is 2 f/s. In this case, x = 2 (f/s)× 150/50 = 6 according to a computation method similar to that of the above-described Formula A to set the amount of the change in the range of interest between the frames to 50. Consequently, 6 f/s is set as the next frame rate. In this case, because the frame rate is 4 f/s at time t1 a, the frame rate changes to 6 f/s at time t1b at which 0.25 sec, which is a period of one frame, has elapsed from time t1a. The frame rate of 6 f/s is maintained until time t2.

Because the area of the range of interest linearly changes at a speed of 500/s during a period of times t2 to t3 (1 sec), the amount of the change in the range of interest immediately after time t2 is 500/6 = 83 when the frame rate is 6 f/s. In this case, x = 6 (f/s)×83/50 = 10 according to a computation method similar to that of the above-described Formula A to set the amount of the change in the range of interest between the frames to 50. Consequently, 10 f/s is set as the next frame rate.

The above-described process is iterated until time t5 at which the range of interest is in the effective imaging region. After time t2, the frame rate is sequentially set to 10 f/s, 14 f/s, and 18 f/s until time t5. While the amount of the change in the range of interest between the frames exceeds 50, which is the reference value, the frame rate is set to a value greater than the frame rate determined in a previous frame.

After time t5, the range of interest gradually deviates from the effective imaging region. In this case, the notification of the contact of the range of interest with an outer frame of the effective image region is provided from the range-of-interest detection unit 15 to the frame rate determination unit 17 and the frame rate is uniformly set to 10 f/s. Thus, the frame rate during a period of times t5 to t6 is set to 10 f/s, which is a predetermined value. Although the frame rate is originally 50 f/s according to computation similar to that described above, the frame rate until the range of interest completely deviates from the effective imaging region is maintained to be 10 f/s when the range-of-interest detection unit 15 detects that the range of interest deviates from the effective imaging region.

After time t6, the range of interest completely deviates from the effective imaging region. When it is confirmed that the range of interest deviates from the effective imaging region in a frame immediately after time t6, the frame rate is set to 2 f/s, which is a predetermined value.

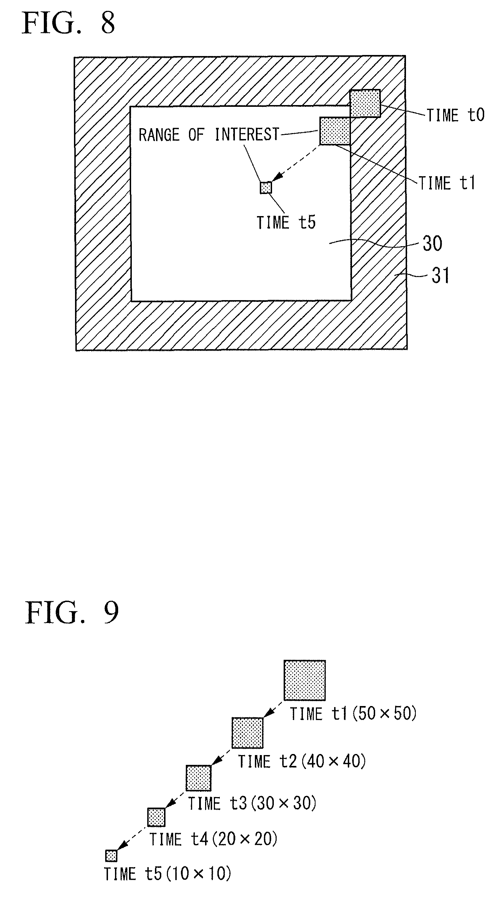

Next, the setting of the frame rate when the traveling direction of the capsule endoscope 1 is reversed (when the capsule endoscope 1 travels backward) will be described using Figs. 8 to 11. Figs. 8 to 11 correspond to Figs. 4 to 7, respectively.

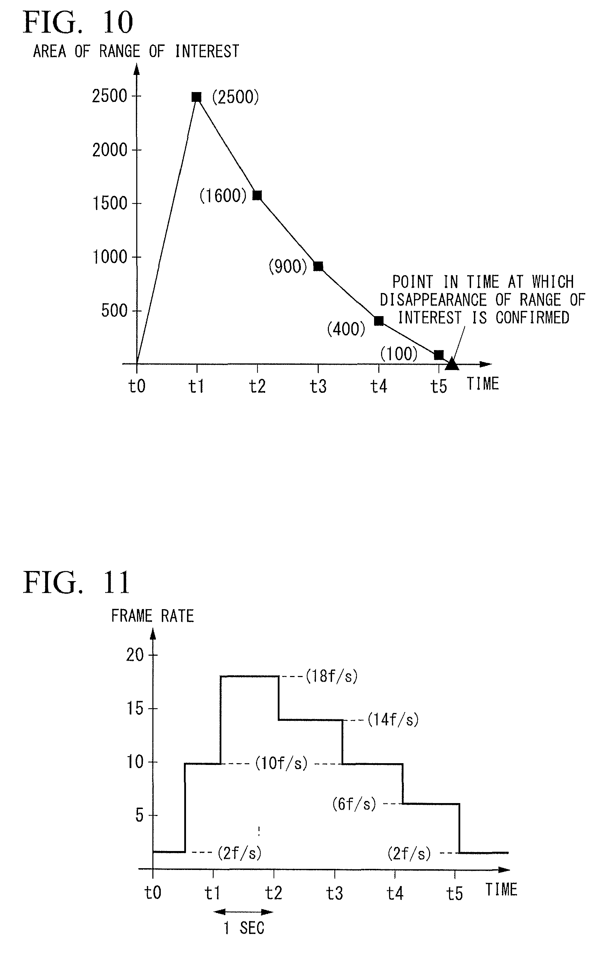

As illustrated in Fig. 8, the range of interest is in the effective imaging region from time t0. At time t1, the entire range of interest is included within the effective imaging region. In this case, the area of the range of interest sequentially changes to 50×50 (t1) → 40×40 (t2) → 30×30 (t3) → 20×20 (t4) → 10×10 (t5) as illustrated in Fig. 9. Fig. 10 illustrates an area of the range of interest at each time and a change in the area between times when the area of the range of interest changes as illustrated in Fig. 9. The horizontal axis of Fig. 10 represents time and the vertical axis of Fig. 10 represents an area of a range of interest. A change in the area between the times is linearly approximated.

Even when the traveling direction of the capsule endoscope 1 is reversed, the following conditions are applied.
(1) The frame rate is 10 f/s when the range of interest is not completely in the effective imaging region.
(2) The reference value of the amount of the change in the range of interest between the frames is 50. The amount of the change is an absolute value and the reference value is 50 even when the area of the range of interest increases or decreases.

Fig. 11 illustrates an example of a frame rate corresponding to the change in the area of the range of interest illustrated in Fig. 10. The horizontal axis of Fig. 11 represents time and the vertical axis of Fig. 11 represents a frame rate (f/s). Before the range of interest is detected, the frame rate is fixed at 2 f/s. Because the range of interest is detected in the captured image of a first frame after time t0 and the range of interest is in contact with the outer frame of the effective imaging region, the frame rate is set to 10 f/s. An example in which the range of interest is first detected at time t0 and the frame rate changes to 10 f/s at a point in time at which 0.5 sec, which is a period of one frame, from time t0 has elapsed is illustrated in Fig. 11.

The detection of the amount of the change in the range of interest in this embodiment is performed using an image for which the entire range of interest is within the effective imaging region. Because the entire range of interest is within the effective imaging region at time t1, a first amount of the change is detected using a captured image of a first frame immediately after time t1 and a captured image of the next frame. The frame rate is set to 10 f/s until then.

Because the amount of the change in the range of interest during a period of times t1 to t2 is 900, the amount of the change in the range of interest between the frames when the frame rate is 10 f/s is 90. A newly set frame rate x is x = 10 (f/s)x90/50 = 18. Consequently, 18 f/s is set as the next frame rate.

Because the amount of the change in the range of interest during a period of times t2 to t3 is 700, the amount of the change in the range of interest between the frames when the frame rate is 18 f/s is 38. A newly set frame rate x is x = 18 (f/s)×38/50 = 14. Consequently, 14 f/s is set as the next frame rate.

In a similar procedure, the frame rate until time t5 is set. After time t2, the frame rate is sequentially set to 18 f/s, 14 f/s, and 10 f/s until time t5. While the amount of the change in the range of interest between the frames is less than 50, which is the reference value, the frame rate is set to a value less than the frame rate determined in a previous frame. When no range of interest is detected after time t5, the frame rate is set to 2 f/s.

### [Details of frame rate-setting process]

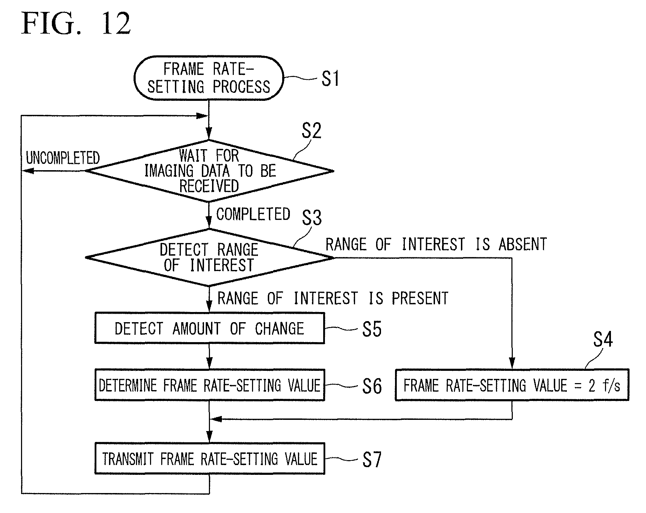

Fig. 12 illustrates a procedure of the frame rate-setting process. When the frame rate-setting process S1 starts, the reception device control unit 18 awaits the reception of imaging data for one frame transmitted from the capsule endoscope 1 (S2). When the imaging data is transmitted from the capsule endoscope 1, the reception device control unit 18 causes the wireless communication unit 10 to receive imaging data. When the wireless communication unit 10 receives imaging data for one frame, the range-of-interest detection unit 15 detects the range of interest in the imaging data based on reference image information stored in the reference image storage unit 14 (S3).

When no range of interest is detected because there is no range of interest, the frame rate determination unit 17 is notified of the fact that there is no range of interest and the frame rate determination unit 17 determines the frame rate-setting value as 2 f/s (S4). The reception device control unit 18 is notified of the determined frame rate-setting value.

When the range of interest is detected, the amount-of-change detection unit 16 is notified of the range of interest along with the fact that the range of interest is present and the amount-of-change detection unit 16 detects an amount of change in the range of interest between imaging data of two different frames (S5). The frame rate determination unit 17 is notified of the detected amount of the change. When the range of interest is present and the range of interest is in contact with the outer frame of the effective imaging region, the frame rate determination unit 17 is notified of the presence and contact of the range of interest and the amount of the change in the range of interest is not detected.

After the amount of the change in the range of interest is detected, the frame rate determination unit 17 determines the frame rate-setting value based on a frame rate-setting value corresponding to a current frame rate determined one frame before and a detected amount of the change (S6). As described above, the frame rate determination unit 17 determines the frame rate-setting value to be transmitted to the capsule endoscope 1 as a value greater than a previously determined frame rate-setting value when the amount of the change exceeds a predetermined reference value. The frame rate determination unit 17 determines the frame rate-setting value to be transmitted to the capsule endoscope 1 as a value less than the previously determined frame rate-setting value when the amount of the change is less than the predetermined reference value. The reception device control unit 18 is notified of the determined frame rate-setting value.

The reception device control unit 18 controls the frame rate-setting value of the notification from the frame rate determination unit 17 to be transmitted to the capsule endoscope 1 (S7). That is, the reception device control unit 18 controls the wireless communication unit 10 to transmit the determined frame rate-setting value to the capsule endoscope 1. After the transmission of the frame rate-setting value, the reception device control unit 18 awaits the reception of imaging data of the next frame (S2).

According to this embodiment, a data reception device is provided (the reception device 3), including: a wireless communication interface (the wireless communication unit 10) configured to receive imaging data transmitted from the capsule endoscope 1 in which a frame rate of imaging is changeable based on a frame rate-setting value for designating the frame rate and which transmits the imaging data after the imaging by performing the imaging and transmit the frame rate-setting value to the capsule endoscope 1; a storage module (the reference image storage unit 14) configured to store reference image information to be used to specify a part of a living body among the imaging data; the range-of-interest detection unit 15 configured to detect a range of interest which is a range of the part in the imaging data based on the reference image information; the amount-of-change detection unit 16 configured to detect an amount of change in the range of interest between the imaging data of two different frames; and the frame rate determination unit 17 configured to determine the frame rate-setting value to be transmitted to the capsule endoscope 1 as a value greater than a previously determined frame rate-setting value when the amount of the change in the range of interest exceeds a predetermined reference value and determine the frame rate-setting value to be transmitted to the capsule endoscope 1 as a value less than the previously determined frame rate-setting value when the amount of the change in the range of interest is less than the predetermined reference value.

According to this embodiment, a capsule endoscope system is providedhaving the capsule endoscope 1 and a data reception device (the reception device 3), wherein the capsule endoscope 1 includes: an imaging module (the image sensor 5) in which a frame rate of imaging is changeable based on a frame rate-setting value for designating the frame rate and configured to output imaging data after the imaging by performing the imaging; and a first wireless communication interface (the wireless communication unit 7 and the antenna unit 8) configured to transmit the imaging data output from the imaging module (the image sensor 5) to the data reception device (the reception device 3) and receive the frame rate-setting value from the data reception device (the reception device 3), and wherein the data reception device (the reception device 3) includes: a second wireless communication interface (the wireless communication unit 10) configured to receive the imaging data from the capsule endoscope 1 and transmit the frame rate-setting value to the capsule endoscope 1; a storage module (the reference image storage unit 14) configured to store reference image information to be used to specify a part of a living body among the imaging data; the range-of-interest detection unit 15 configured to detect a range of interest which is a range of the part in the imaging data based on the reference image information; the amount-of-change detection unit 16 configured to detect an amount of change in the range of interest between the imaging data of two different frames; and the frame rate determination unit 17 configured to determine the frame rate-setting value to be transmitted to the capsule endoscope 1 as a value greater than a previously determined frame rate-setting value when the amount of the change in the range of interest exceeds a predetermined reference value and determine the frame rate-setting value to be transmitted to the capsule endoscope 1 as a value less than the previously determined frame rate-setting value when the amount of the change in the range of interest is less than the predetermined reference value.

According to this embodiment, a data reception method is provided, including: the step S2 of receiving, by a wireless communication interface (the wireless communication unit 10), imaging data transmitted from the capsule endoscope 1 in which a frame rate of imaging is changeable based on a frame rate-setting value for designating the frame rate and which transmits imaging data after the imaging by performing the imaging; the step S3 of detecting, by the range-of-interest detection unit 15, a range of interest which is a range of a part of a living body in the imaging data based on reference image information of a storage module (the reference image storage unit 14) configured to store the reference image information to be used to specify the part among the imaging data; the step S5 of detecting, by the amount-of-change detection unit 16, an amount of change in the range of interest between the imaging data of two different frames; the step S6 of determining, by the frame rate determination unit 17, the frame rate-setting value to be transmitted to the capsule endoscope 1 as a value greater than a previously determined frame rate-setting value when the amount of the change in the range of interest exceeds a predetermined reference value and determining the frame rate-setting value to be transmitted to the capsule endoscope 1 as a value less than the previously determined frame rate-setting value when the amount of the change in the range of interest is less than the predetermined reference value; and the step S7 of transmitting, by the wireless communication interface (the wireless communication unit 10), the determined frame rate-setting value to the capsule endoscope 1.

According to this embodiment, a program is provided for causing a computer to execute: the step S2 of causing a wireless communication interface (the wireless communication unit 10) to receive imaging data transmitted from the capsule endoscope 1 in which a frame rate of imaging is changeable based on a frame rate-setting value for designating the frame rate and which transmits imaging data after the imaging by performing the imaging; the step S3 of detecting a range of interest which is a range of a part of a living body in the imaging data based on reference image information of a storage module (the reference image storage unit 14) configured to store the reference image information to be used to specify the part among the imaging data; the step S5 of detecting an amount of change in the range of interest between the imaging data of two different frames; the step S6 of determining the frame rate-setting value to be transmitted to the capsule endoscope 1 as a value greater than a previously determined frame rate-setting value when the amount of the change in the range of interest exceeds a predetermined reference value and determining the frame rate-setting value to be transmitted to the capsule endoscope 1 as a value less than the previously determined frame rate-setting value when the amount of the change in the range of interest is less than the predetermined reference value; and the step S7 of causing the wireless communication interface (the wireless communication 10) to transmit the determined frame rate-setting value to the capsule endoscope 1.

In this embodiment, it is possible to maintain a high imaging speed of the capsule endoscope system according to the present invention when a change in a captured image is large because the frame rate-setting value is determined to be a value greater than the previous frame rate-setting value when the amount of the change in the range of interest exceeds a predetermined reference value. Also, in this embodiment, it is possible to reduce power consumption of the capsule endoscope system according to the present invention when the amount of the change in a captured image is small because the frame rate-setting value is determined to be a value less than the previous frame rate-setting value when the amount of the change in the range of interest exceeds the predetermined reference value.

According to this embodiment, a data reception device (the reception device 3) is provided having a storage module (the reference image storage unit 14) configured to store a plurality of pieces of reference image information, wherein the reference value is set to be changeable for each piece of the reference image information. Thereby, the capsule endoscope system according to the present invention can perform imaging at a suitable frame rate according to a lesion part. For example, even when the lesion part which is an important observation target is small and the amount of the change in the range of interest is small, the capsule endoscope system according to the present invention can perform imaging at an increased frame rate.

### (Second embodiment)

Next, the second embodiment of the present invention will be described. The configuration of this embodiment is similar to the configuration described in the first embodiment. Some operations of the frame rate-setting process in this embodiment are different from the operations described in the first embodiment.

The capsule endoscope system according to this embodiment has a function of constantly setting a frame rate of the case in which a range of interest is in a captured image to a value greater than a frame rate of the case in which no range of interest is in the captured image. Specifically, a frame rate-setting value of the case in which no range of interest is in the captured image is constantly 2 f/s and a lower limit value of a frame rate-setting value of the case in which the range of interest is in the captured image is 4 f/s.

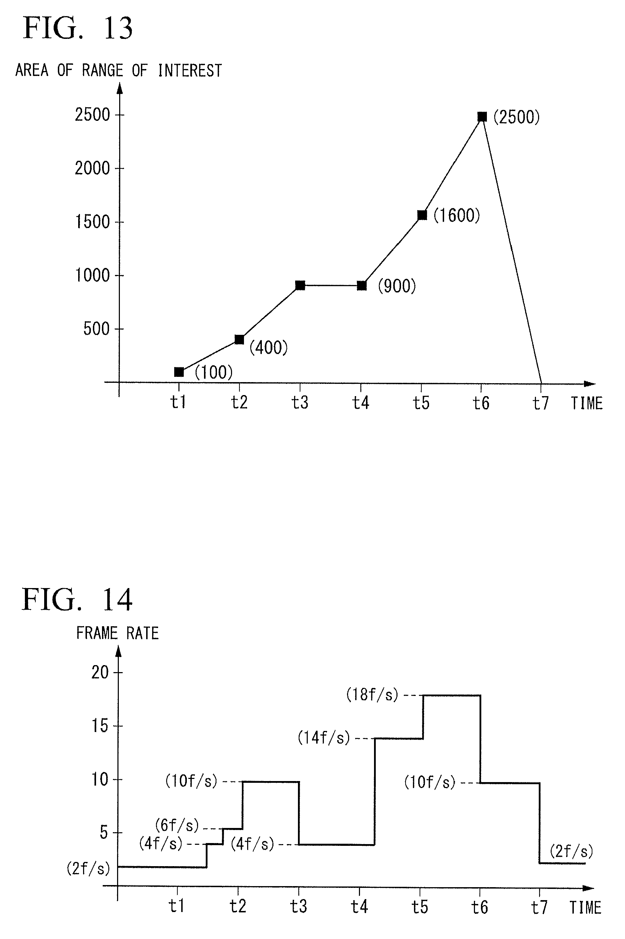

Fig. 13 illustrates an area of the range of interest at each time and a change in the area between times. The horizontal axis of Fig. 13 represents time and the vertical axis of Fig. 13 represents an area of a range of interest. A change in the area between the times is linearly approximated. Fig. 14 illustrates an example of the frame rate corresponding to the change in the area of the range of interest illustrated in Fig. 13. The horizontal axis of Fig. 14 represents time and the vertical axis of Fig. 14 represents a frame rate (f/s).

Although a change in an area of a range of interest in this embodiment is similar to the change in the area of the range of interest illustrated in Fig. 5 in the first embodiment, this embodiment is different from the example described in the first embodiment in that a capsule endoscope 1 is static for 1 sec immediately after time t3.

The change in the frame rate until time t3 is similar to the change in the frame rate described in the first embodiment. A state in which the capsule endoscope 1 is static for 1 sec immediately after time t3 and the area of the range of interest does not change while the capsule endoscope 1 is static is illustrated in Fig. 13. When the capsule endoscope 1 is static, the amount of the change in the range of interest is zero. When the amount of the change in the range of interest is zero, the frame rate-setting value is determined to be a lower limit value.

Because the amount of the change in an image of interest detected immediately after time t3 is zero, a computed value of the next set frame rate is x = 0 as illustrated in Fig. 14. Because the computed frame rate-setting value is less than a lower limit value 4 f/s of the frame rate-setting value of the case in which there is a range of interest, the frame rate-setting value is determined to be the lower limit value 4 f/s of the frame rate-setting value. Until time t4 at which the capsule endoscope 1 resumes movement, the frame rate is set to 4 f/s as illustrated in Fig. 14.

When the capsule endoscope 1 resumes movement at time t4, the amount of the change in the range of interest between the frames when the frame rate is 4 f/s is 175 because the amount of the change in the range of interest during a period of times t4 to t5 is 700. A newly set frame rate x is x = 4 (f/s)×175/50 = 14. Consequently, 14 f/s is set as the next frame rate. The change in the subsequent frame rate is similar to the change in the frame rate described in the first embodiment.

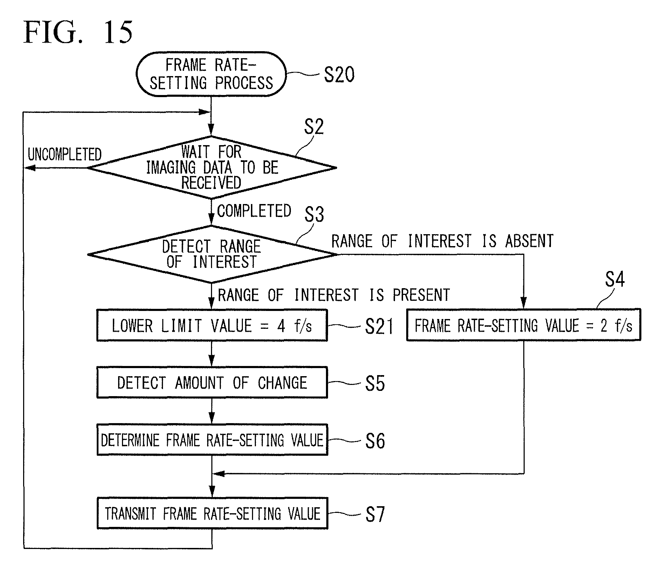

Fig. 15 illustrates a procedure of a frame rate-setting process. A frame rate-setting process S20 illustrated in Fig. 15 is different from the frame rate-setting process S1 illustrated in Fig. 12 in that a process of setting a lower limit value of a frame rate-setting value is performed when a range of interest is detected.

When the range of interest is detected, a frame rate determination unit 17 is notified of the fact that the range of interest is present. The frame rate determination unit 17 internally holds the lower limit value of the frame rate-setting value (S21). The lower limit value 4 f/s of the frame rate-setting value is a value greater than the frame rate-setting value 2 f/s when no range of interest is detected. After the detection (S5) of the amount of the change in the range of interest is performed, the frame rate-setting value is controlled not to be less than the lower limit value when the frame rate determination unit 17 determines the frame rate-setting value (S6).

That is, the frame rate determination unit 17 determines the frame rate-setting value as a predetermined value 2 f/s when no range of interest is detected. When the range of interest is detected, the frame rate determination unit 17 sets a lower limit value greater than the predetermined value 2 f/s. Further, the frame rate determination unit 17 determines the frame rate-setting value according to an amount of change in the range of interest as a value greater than or equal to the lower limit value.

Because a process other than the above-described process is similar to that performed in the frame rate-setting process S1 illustrated in Fig. 12, description thereof will be omitted.

According to this embodiment, a data reception device (a reception device 3) having the frame rate determination unit 17 is configured. When no range of interest is detected, the frame rate determination unit 17 determines the frame rate-setting value to be transmitted to the capsule endoscope 1 as a predetermined value. When the range of interest is detected, the frame rate determination unit 17 sets a lower limit value greater than the predetermined value and determines the frame rate-setting value to be transmitted to the capsule endoscope 1 according to the amount of the change in the range of interest as a value greater than or equal to the lower limit value.

Thereby, the capsule endoscope 1 stops during imaging of the lesion part. Thereafter, when the movement starts, the capsule endoscope 1 can shorten the delay of detection of the lesion part immediately after the start of movement.

### (Third embodiment)

Next, the third embodiment of the present invention will be described. The configuration of this embodiment is similar to the configuration described in the first embodiment. Some operations of a frame rate-setting process in this embodiment are different from the operations described in the first embodiment.

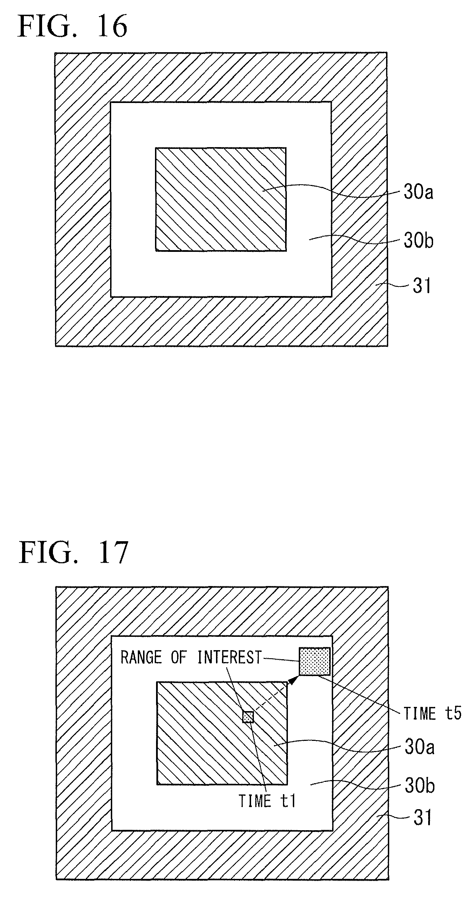

In this embodiment, a frame rate is set in accordance with a position in a captured image from which a range of interest is discovered when the range of interest is first discovered. Fig. 16 illustrates a range of imaging data to be transmitted from a capsule endoscope 1 to a reception device 3. An effective imaging region includes an auxiliary imaging region 30a in which a relatively distant view (a first imaging target) is imaged and a main imaging region 30b in which a relatively close view (a second imaging target of a closer view than the first imaging target of the auxiliary imaging region 30a) is imaged. The auxiliary imaging region 30a is a rectangular region which is a part of an image (an image of the effective imaging region) based on the imaging data and includes the center of the image. The main imaging region 30b is a region which is a part of an image (an image of the effective imaging region) based on the imaging data and outside the auxiliary imaging region 30a.

In this embodiment, the frame rate is set as follows.
(1) When a position at which the range of interest is first discovered is in the auxiliary imaging region 30a, the frame rate is set to 15 f/s.
(2) When the position at which the range of interest is first discovered is in the main imaging region 30b, the frame rate is set to 30 f/s.
(3) A reference value, which is a target value of the amount of the change in the range of interest between frames is 50, which is equal to the reference value of the first embodiment when the range of interest is in the auxiliary imaging region 30a.
(4) The reference value is 25 when the range of interest is in the main imaging region 30b.

That is, the frame rate-setting value of the case in which the range of interest is in the main imaging region 30b is set to be twice the frame rate-setting value of the case in which the range of interest is in the auxiliary imaging region 30a. The above-described specific numerical value is an example and the present invention is not limited thereto.

The amount of the change in the range of interest between frames is not accurately known at a point in time at which the range of interest is first discovered. Thus, the frame rate-setting value is set to a fixed value to prevent a wrong frame rate from being set. If imaging is not immediately performed when the position at which the range of interest is first discovered is in the main imaging region 30b, a chance to capture a desired image is likely to be missed. Thus, the frame rate-setting value of the case in which the position at which the range of interest is first discovered is in the main imaging region 30b is set to a value greater than the frame rate-setting value of the case in which the position at which the range of interest is first discovered is in the auxiliary imaging region 30a.

The main imaging region 30b is a region in which an image is captured when a lesion part is in the vicinity of the capsule endoscope 1. Thus, in the main imaging region 30b, the resolution is high and an image effective for the diagnosis is likely to be captured. Consequently, in order to capture more images when the range of interest is in the main imaging region 30b, the frame rate-setting value is set to a larger value.

Fig. 17 illustrates an example of movement of the range of interest when the range of interest is discovered in the auxiliary imaging region 30a and moves outside an imaging range through the main imaging region 30b. The range of interest is initially in the auxiliary imaging region 30a at time t1 and the range of interest moves to the main imaging region 30b when a time has elapsed. The range of interest is positioned at an end of the main imaging region 30b at time t5.

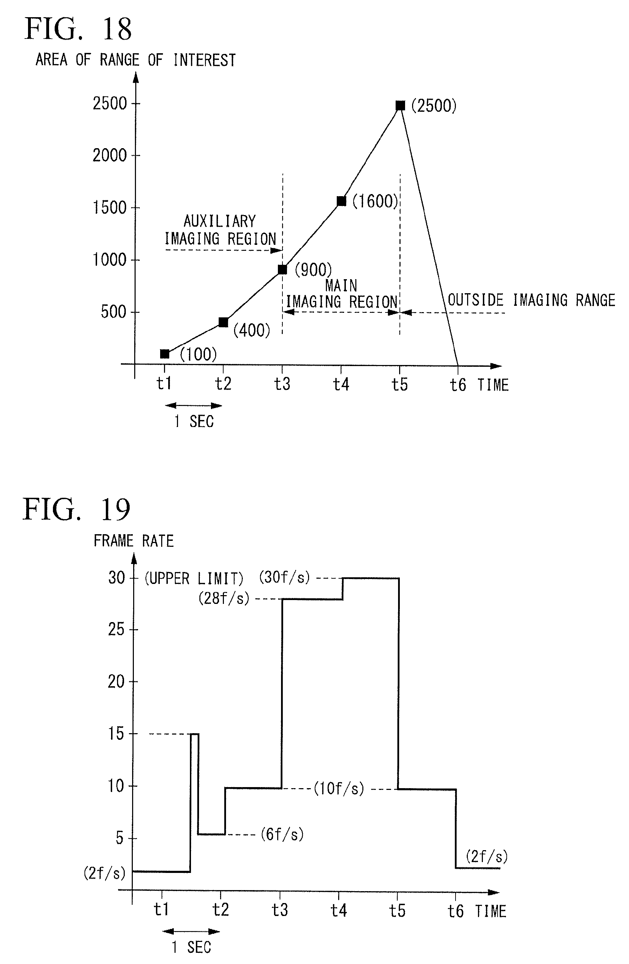

Fig. 18 illustrates an area of the range of interest at each time and a change in the area between times. The horizontal axis of Fig. 18 represents time and the vertical axis of Fig. 18 represents an area of a range of interest. A change in the area between the times is linearly approximated. The range of interest is in the auxiliary imaging region 30a during a period of times t1 to t3 and the range of interest is in the main imaging region 30b during a period of times t3 to t5.

Fig. 19 illustrates an example of a frame rate corresponding to the change in the area of the range of interest illustrated in Fig. 18. The horizontal axis of Fig. 19 represents time and the vertical axis of Fig. 19 represents a frame rate (f/s). The setting of the frame rate will be described using Fig. 19.

Before the range of interest is detected, the frame rate is fixed at 2 f/s. Because the range of interest is first discovered in the auxiliary imaging region 30a at time t1, the frame rate is set to 15 f/s at a point in time at which a period of one frame has elapsed from time t1.

A reference value is 50, which is equal to the reference value of the first embodiment, because the range of interest during a period of times t1 to t3 is in the auxiliary imaging region 30a. Thus, the frame rate immediately after being set to 15 f/s is set to 6 f/s, which is equal to that of the first embodiment.

Because the range of interest during a period of times t3 to t5 is in the main imaging region 30b, the reference value is 25. Because the amount of the change in the range of interest during a period of times t3 to t4 is 700, the amount of the change in the range of interest between the frames when the frame rate is 6 f/s is 117. A newly set frame rate x is x = 6 (f/s)×117/25 = 28. Consequently, 28 f/s is set as the next frame rate.

Because the amount of the change in the range of interest during a period of times t4 to t5 is 900, the amount of the change in the range of interest between the frames when the frame rate is 28 f/s is 32. A newly set frame rate x is x = 28 (f/s)x32/25 = 36. Because an upper limit value of the frame rate is 30 f/s, 30 f/s is set as the next frame rate.

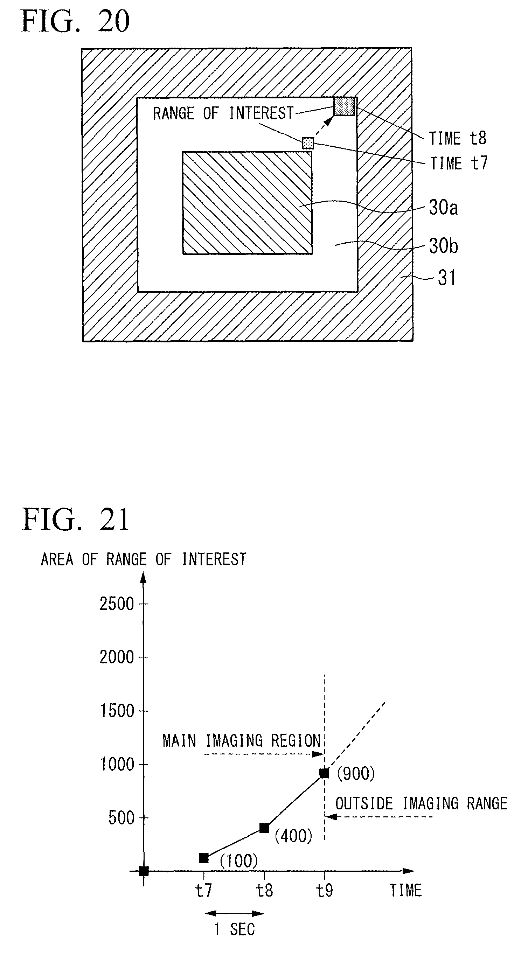

Fig. 20 illustrates an example of movement of a range of interest when the range of interest is discovered in the main imaging region 30b and moves to a region 31 outside an imaging range. The range of interest is initially discovered in the main imaging region 30b at time t7 and the range of interest moves toward the region 31 outside the imaging range when a time has elapsed. The range of interest is positioned at an end of the main imaging region 30b at time t8.

Fig. 21 illustrates an area of the range of interest at each time and a change in the area between times. The horizontal axis of Fig. 21 represents time and the vertical axis of Fig. 21 represents an area of a range of interest. A change in the area between the times is linearly approximated. As illustrated in Fig. 21, the area of the range of interest at time t7 is 100, the area of the range of interest at time t8 is 400, and the area of the range of interest at time t9 is 900. The range of interest is in the main imaging region 30b during a period of times t7 to t9. A change in the area of the range of interest during a period of times t7 to t9 is the same as the change in the area of the range of interest during a period of times t1 to t3 illustrated in Fig. 6 of the first embodiment.

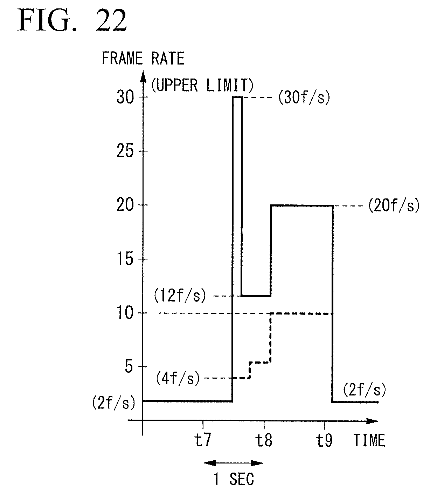

Fig. 22 illustrates an example of a frame rate corresponding to the change in the area of the range of interest illustrated in Fig. 21. In Fig. 22, the horizontal axis represents time and the vertical axis represents a frame rate (f/s). The setting of the frame rate will be described using Fig. 22.

Before the range of interest is detected, the frame rate is fixed at 2 f/s. Because the range of interest is first discovered in the main imaging region 30b at time t7, the frame rate is set to 30 f/s at a point in time at which a period of one frame has elapsed from time t7.

Because the range of interest during a period of times t7 to t9 is in the main imaging region 30b, the reference value is 25. Because the amount of the change in the range of interest during a period of times t7 to t8 is 300, the amount of the change in the range of interest between the frames when the frame rate is 30 f/s is 10. A newly set frame rate x is x = 30 (f/s)×10/25 = 12. Consequently, 12 f/s is set as the next frame rate.

Because the amount of the change in the range of interest during a period of times t8 to t9 is 500, the amount of the change in the range of interest between the frames when the frame rate is 12 f/s is 42. A newly set frame rate x is x = 12 (f/s)×42/25 = 20. Consequently, 20 f/s is set as the next frame rate.

A broken line of Fig. 22 indicates a value of a frame rate computed by the method shown in the first embodiment. Because the reference value of the case in which the range of interest is in the main imaging region 30b in the third embodiment is half the reference value in the first embodiment, the frame rate in the third embodiment is twice the frame rate computed by the method shown in the first embodiment. The broken line of Fig. 22 also matches the frame rate computed when the range of interest is in the auxiliary imaging region 30a. Accordingly, when the range of interest is in the main imaging region 30b, the frame rate is set to be greater than the frame rate of the case in which the range of interest is in the auxiliary imaging region 30a.

In order to implement the above-described frame rate setting, a range-of-interest detection unit 15 determines whether the range of interest is located in the auxiliary imaging region 30a in which a relatively distant view is imaged or the main imaging region 30b in which a relatively close view is imaged when the range of interest is detected. When it is determined that the range of interest is located in the main imaging region 30b, a frame rate determination unit 17 determines the frame rate-setting value to be transmitted to the capsule endoscope 1 according to the amount of the change in the range of interest as a value greater than the frame rate-setting value determined when it is determined that the range of interest is located in the auxiliary imaging region 30a.

Further, when the range of interest is first detected after a point in time at which the detection of the range of interest starts, the frame rate determination unit 17 determines the frame rate-setting value to be transmitted to the capsule endoscope 1 as 15 f/s when it is determined that the detected range of interest is located in the auxiliary imaging region 30a. The frame rate determination unit 17 determines the frame rate-setting value to be transmitted to the capsule endoscope 1 as 30 f/s, which is greater than 15 f/s, when it is determined that the detected range of interest is located in the main imaging region 30b.

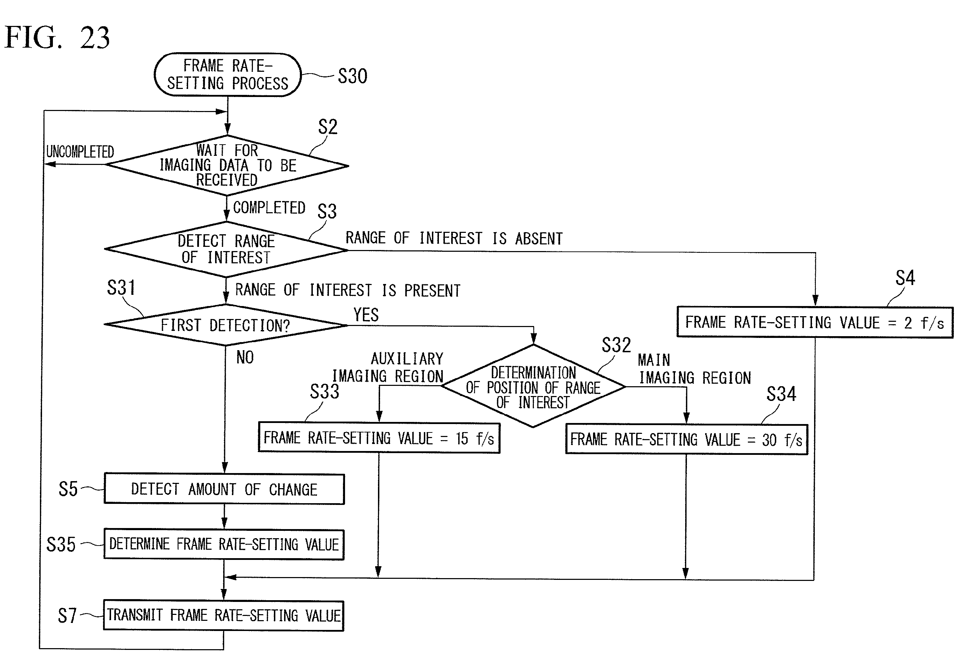

Fig. 23 illustrates a procedure of the frame rate-setting process. The frame rate-setting process S30 illustrated in Fig. 23 is different from the frame rate-setting process S 1 illustrated in Fig. 12 in that a process of changing the frame rate-setting value according to a detection position is performed when the range of interest is first detected.

When the range of interest is detected, a range-of-interest detection unit 15 determines whether the range of interest is first detected after a point in time at which the frame rate-setting process starts (whether the detection of the range of interest of the current time is first detection) (S31). When the range of interest is already detected and the current detection is a second or subsequent detection (when the range of interest is detected twice or more in a detection process including detection of the current time), an amount-of-change detection unit 16 is notified of the range of interest. The amount-of-change detection unit 16 detects the amount of the change in the range of interest between imaging data of two different frames (S5).

When the range of interest is first detected after the point in time at which the frame rate-setting process starts (when the detection of the range of interest of the current time is the first detection), the range-of-interest detection unit 15 determines whether the detected range of interest is located in the auxiliary imaging region 30a or the main imaging region 30b (S32). The frame rate determination unit 17 is notified of a determination result.

When it is determined that the detected range of interest is located in the auxiliary imaging region 30a, the frame rate determination unit 17 determines the frame rate-setting value to be transmitted to the capsule endoscope 1 as 15 f/s (S33). When it is determined that the detected range of interest is located in the main imaging region 30b, the frame rate determination unit 17 determines the frame rate-setting value to be transmitted to the capsule endoscope 1 as 30 f/s (S34).

When the range of interest is detected twice or more after a point in time at which the frame rate-setting process starts, the amount-of-change detection unit 16 detects the amount of the change in the range of interest in S5. Thereafter, the frame rate determination unit 17 determines the frame rate-setting value based on the frame rate-setting value corresponding to a current frame rate determined one frame before and the detected amount of the change (S35). As described above, when the amount of the change in the range of interest exceeds the reference value, the frame rate determination unit 17 determines the frame rate-setting value to be transmitted to the capsule endoscope 1 as a value greater than a previously determined frame rate-setting value. When the amount of the change in the range of interest is less than the reference value, the frame rate determination unit 17 determines the frame rate-setting value to be transmitted to the capsule endoscope 1 as a value less than the previously determined frame rate-setting value.

As described above, the reference value is 25 when the range of interest is in the main imaging region 30b and the reference value is 50 when the range of interest is in the auxiliary imaging region 30a. Thus, the frame rate-setting value of the case in which the range of interest is in the main imaging region 30b is set to be greater than the frame rate-setting value of the case in which the range of interest is in the auxiliary imaging region 30a. The frame rate-setting value is a value according to an amount of change detected by the amount-of-change detection unit 16.

In the above-described manner, a reception device control unit 18 is notified of the frame rate-setting value determined in S33, S34, and S35. Because a process other than the above-described process is similar to that performed in the frame rate-setting process S1 illustrated in Fig. 12, description thereof will be omitted.

According to this embodiment, a data reception device (the reception device 3) having the range-of-interest detection unit 15 and the frame rate determination unit 17 is configured. The range-of-interest detection unit 15 determines whether the range of interest is located in a first region (the auxiliary imaging region 30a) in which a relatively distant view is imaged or a second region (the main imaging region 30b) in which a relatively close view is imaged when the range of interest is detected. When it is determined that the range of interest is located in the second region (the main imaging region 30b), the frame rate determination unit 17 determines the frame rate-setting value to be transmitted to the capsule endoscope 1 according to the amount of the change in the range of interest as a value greater than the frame rate-setting value determined when it is determined that the range of interest is located in the first region (the auxiliary imaging region 30a).

According to this embodiment, the data reception device (the reception device 3) in which the first region (the auxiliary imaging region 30a) is a region which is a part of an image based on the imaging data and includes a center of the image is configured.

In the second region (the main imaging region 30b), the resolution is high and an image effective for the diagnosis is likely to be captured. Thus, more effective images necessary for the diagnosis can be captured by determining the frame rate-setting value as a larger value when the range of interest is in the second region (the main imaging region 30b).

According to this embodiment, a data reception device (the reception device 3) having the frame rate determination unit 17 is configured. When the range of interest is first detected after a point in time at which the detection of the range of interest starts, the frame rate determination unit 17 determines the frame rate-setting value to be transmitted to the capsule endoscope 1 as a first predetermined value when it is determined that the detected range of interest is located in the first region (the auxiliary imaging region 30a). The frame rate determination unit 17 determines the frame rate-setting value to be transmitted to the capsule endoscope 1 as a second predetermined value greater than the first predetermined value when it is determined that the detected range of interest is located in the second region (the main imaging region 30b).

At a point in time at which the range of interest is first discovered, the frame rate determination unit 17 determines the frame rate-setting value of the case in which the range of interest is in the second region (the main imaging region 30b) as a value greater than the frame rate-setting value of the case in which the range of interest is in the first region (the auxiliary imaging region 30a). Thereby, it is possible to immediately capture an image necessary for the diagnosis.

### (Fourth embodiment)

Next, the fourth embodiment of the present invention will be described. The capsule endoscope system of this embodiment sets a frame rate using a change in imaging data of a range of interest and a background outside the range of interest between frames. The background is a region outside the range of interest when the range of interest is detected and is the entire effective imaging region when no range of interest is detected.

In the detection of a change in an image of a background in this embodiment, a change in a color tone of the background or a change in a main shape is detected. Because it is recognized whether a situation of an image of a joint of an organ or the like largely changes from a change in a background image, the frame rate is controlled so that the frame rate increases immediately after the change.

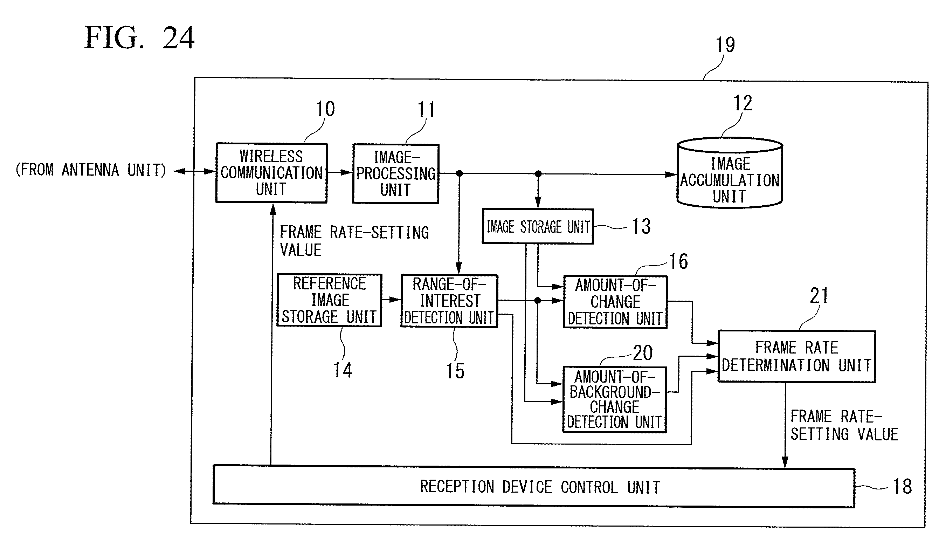

The configuration of this embodiment is similar to the configuration described in the first embodiment, except that the reception device 3 described in the first embodiment is replaced with a reception device 19 illustrated in Fig. 24. As illustrated in Fig. 24, the reception device 19 has a wireless communication unit 10, an image-processing unit 11, an image accumulation unit 12, an image storage unit 13, a reference image storage unit 14, a range-of-interest detection unit 15, an amount-of-change detection unit 16, a reception device control unit 18, an amount-of-background-change detection unit 20, and a frame rate determination unit 21.

Information about the range of interest is input from the range-of-interest detection unit 15 to the amount-of-background-change detection unit 20 and imaging data of a current frame and imaging data of a previous frame are input from the image storage unit 13. The amount-of-background-change detection unit 20 detects an amount of background change, which is an amount of change in a background outside the range of interest between imaging data of two different frames, and the detected amount of the background change is output to the frame rate determination unit 21.

The frame rate determination unit 21 determines the frame rate-setting value to be transmitted to the capsule endoscope 1 as a value according to the amount of the change detected by the amount-of-change detection unit 16 and the amount of the background change detected by the amount-of-background-change detection unit 20. The frame rate determination unit 21 outputs the determined frame rate-setting value to the reception device control unit 18. More specifically, when the range of interest is detected, the frame rate determination unit 21 determines the frame rate-setting value to be transmitted to the capsule endoscope 1 according to the amount of the change as a value reflecting the amount of the background change at a first degree of influence. When no range of interest is detected, the frame rate determination unit 21 determines the frame rate-setting value to be transmitted to the capsule endoscope 1 as a value reflecting the amount of the background change at a second degree of influence lower than the first degree of influence. The frame rate determination unit 21 internally holds the determined frame rate-setting value. The reception device control unit 18 is notified of the determined frame rate-setting value.

In a state in which no range of interest is detected, the frame rate-setting value is controlled by the amount of the background change. For example, in the first embodiment, the frame rate when no range of interest is detected is 2 f/s. On the other hand, in the fourth embodiment, the frame rate increases according to an excess amount when the amount of the background change exceeds a predetermined value and the amount of the background change is controlled to be within the predetermined value. Also, when the amount of the background change is less than the predetermined value, the frame rate is held to be 2 f/s.

In a state in which the range of interest is detected, both the amount of the change in the range of interest and the amount of the background change are used in setting of the frame rate. Thus, when the amount of the background change is large, a frame rate greater than a frame rate set based on only the amount of the change in the range of interest is set.

When the background changes during the detection of the range of interest, a value for diagnosis of a captured image is large even when no range of interest is detected. Accordingly, because imaging is performed at a high frame rate when the background changes in a state in which the range of interest is detected, a degree of influence of the amount of the background change on the frame rate-setting value is set to be greater than a degree of influence of the case in which no range of interest is detected.

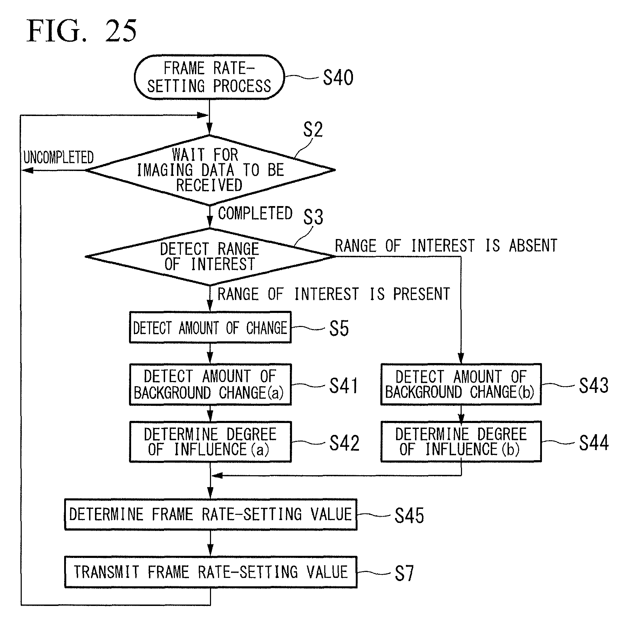

Fig. 25 illustrates a procedure of the frame rate-setting process. The frame rate-setting process S40 illustrated in Fig. 25 is different from the frame rate-setting process S 1 illustrated in Fig. 12 in that the amount of the background change is detected after the detection of the range of interest, the degree of influence of the frame rate of the detected amount of the background change is set after the detection of the amount of the background change, and the frame rate is determined using both the amount of the change in the range of interest and the amount of the background change.

The amount-of-change detection unit 16, the amount-of-background-change detection unit 20, and the frame rate determination unit 21 are notified of the presence/absence of the range of interest in S3. Also, when the range of interest is detected in S3, the amount-of-change detection unit 16 and the amount-of-background-change detection unit 20 are notified of the range of interest.

When the range of interest is detected, the amount-of-change detection unit 16 detects the amount of the change in the range of interest. Thereafter, the amount-of-background-change detection unit 20 recognizes the range of interest and the background based on the range of interest of the notification from the range-of-interest detection unit 15 and detects the amount of the background change between imaging data of a current frame and imaging data of a previous frame (S41). The frame rate determination unit 21 is notified of the detected amount of the background change. After the amount of the background change is detected, the frame rate determination unit 21 determines a degree of influence to be given to the frame rate-setting value (S42). The degree of influence of the case in which the range of interest is detected is determined to be a value greater than a degree of influence of the case in which no range of interest is detected.

On the other hand, when no range of interest is detected, the amount-of-background-change detection unit 20 recognizes that the entire effective imaging region is the background and detects an amount of background change between imaging data of a current frame and imaging data of a previous frame (S43). The frame rate determination unit 21 is notified of the detected amount of the background change. After the amount of the background change is detected, the frame rate determination unit 21 determines a degree of influence to be given to the frame rate-setting value (S44).

When the range of interest is detected, the frame rate determination unit 21 determines the frame rate-setting value according to an amount of change of the range of interest as a value reflecting the amount of the background change at the degree of the influence determined in S42 (S45). For example, the frame rate determination unit 21 determines the frame rate-setting value to be transmitted to the capsule endoscope 1 as a value greater than a previously set frame rate-setting value when an average value between the amount of the change in the range of interest and a value obtained by multiplying the amount of the background change by the degree of the influence exceeds the reference value (for example, 50). When the average value is less than the reference value, the frame rate determination unit 21 determines the frame rate-setting value to be transmitted to the capsule endoscope 1 as a value less than the previously set frame rate-setting value. That is, in this embodiment, computation similar to that described in the first embodiment is performed after the amount of the change in the range of interest in the first embodiment is replaced with the above-described average value.

The reception device control unit 18 is notified of the frame rate-setting value determined in the above-described manner. Because a process other than the above-described process is similar to that performed in the frame rate-setting process S1 illustrated in Fig. 12, description thereof will be omitted.

According to this embodiment, a data reception device (the reception device 3) having the amount-of-background-change detection unit 20 and the frame rate determination unit 21 is configured. The amount-of-background-change detection unit 20 detects the amount of the change in the background outside the range of interest between imaging data of two different frames. The frame rate determination unit 21 determines a frame rate-setting value to be transmitted to the capsule endoscope 1 as a value according to the amount of the change in the range of interest and the amount of the change in the background. Thereby, it is possible to set a frame rate according to the amount of the change in the range of interest and the amount of the change in the background.

According to this embodiment, a data reception device (the reception device 3) having the frame rate determination unit 21 is configured. The frame rate determination unit 21 determines the frame rate-setting value to be transmitted to the capsule endoscope 1 according to the amount of the change in the range of interest as a value reflecting the amount of the change in the background at a first degree of influence when the range of interest is detected. When no range of interest is detected, the frame rate determination unit 21 determines the frame rate-setting value to be transmitted to the capsule endoscope 1 as a value reflecting the amount of the change in the background at a second degree of influence less than the first degree of the influence. Thereby, it is possible to perform imaging at a high frame rate when the background changes in a state in which the range of interest is detected.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

### [Industrial Applicability]

The above-described embodiments can provide a data reception device, a capsule endoscope system, a data reception method, and a program for reducing power consumption while maintaining a high imaging speed according to necessity

### [Reference Signs List]

1 Capsule endoscope
2 Antenna unit
3, 19 Reception device
4 Lens
5 Image sensor
6, 11 Image-processing unit
7, 10 Wireless communication unit
8 Antenna unit
9 Capsule control unit
12 Image accumulation unit
13 Image storage unit
14 Reference image storage unit
15 Range-of-interest detection unit
16 Amount-of-change detection unit
17, 21 Frame rate determination unit
18 Reception device control unit
20 Amount-of background-change detection unit

## Claims

1. A data reception device, comprising:
a wireless communication interface configured to receive imaging data transmitted from a capsule endoscope in which a frame rate of imaging is changeable based on a frame rate-setting value for designating the frame rate and which transmits the imaging data after the imaging by performing the imaging and to transmit the frame rate-setting value to the capsule endoscope;
a storage module configured to store reference image information to be used to specify a part of a living body included in the imaging data;
a range-of-interest detection unit configured to detect a range of interest which is a range of the part in the imaging data based on the reference image information;
an amount-of-change detection unit configured to detect an amount of change in the range of interest between the imaging data of two different frames; and
a frame rate determination unit configured to determine the frame rate-setting value to be transmitted to the capsule endoscope as a value greater than a previously determined frame rate-setting value when the amount of the change in the range of interest exceeds a predetermined reference value, and to determine the frame rate-setting value to be transmitted to the capsule endoscope as a value less than the previously determined frame rate-setting value when the amount of the change in the range of interest is less than the predetermined reference value.

2. The data reception device according to Claim 1, wherein the frame rate determination unit determines the frame rate-setting value to be transmitted to the capsule endoscope as a predetermined value when no range of interest is detected, sets a lower limit value greater than the predetermined value when the range of interest is detected, and determines the frame rate-setting value to be transmitted to the capsule endoscope as a value which corresponds to the amount of the change in the range of interest and being greater than or equal to the lower limit value.

3. The data reception device according to Claim 1,
wherein the range-of-interest detection unit further determines whether the range of interest is located in a first region in which a relatively distant view is imaged or a second region in which a relatively close view is imaged when the range of interest is detected, and
wherein, when it is determined that the range of interest is located in the second region, the frame rate determination unit further determines the frame rate-setting value to be transmitted to the capsule endoscope as a value which corresponds to the amount of the change in the range of interest and being greater than the frame rate-setting value determined when it is determined that the range of interest is located in the first region.

4. The data reception device according to Claim 3, wherein the first region is a region which is a part of an image based on the imaging data and includes a center of the image.

5. The data reception device according to Claim 3, wherein, when the range of interest is first detected after a point in time at which the detection of the range of interest starts, the frame rate determination unit determines the frame rate-setting value to be transmitted to the capsule endoscope as a first predetermined value when it is determined that the detected range of interest is located in the first region, and to determine the frame rate-setting value to be transmitted to the capsule endoscope as a second predetermined value greater than the first predetermined value when it is determined that the detected range of interest is located in the second region.

6. The data reception device according to Claim 1, further comprising:
an amount-of-background-change detection unit configured to detect an amount of change in a background except the range of interest between the imaging data of the two different frames,
wherein the frame rate determination unit determines the frame rate-setting value to be transmitted to the capsule endoscope as a value corresponding to the amount of the change in the range of interest and the amount of the change in the background.

7. The data reception device according to Claim 6, wherein the frame rate determination unit determines the frame rate-setting value to be transmitted to the capsule endoscope corresponding to the amount of the change in the range of interest as a value reflecting the amount of the change in the background at a first degree of influence when the range of interest is detected, and to determine the frame rate-setting value to be transmitted to the capsule endoscope as a value reflecting the amount of the change in the background at a second degree of influence less than the first degree of the influence when no range of interest is detected.

8. The data reception device according to Claim 1,
wherein the storage module stores a plurality of pieces of the reference image information, and
wherein the reference value is set to be changeable for each piece of the reference image information.

9. A capsule endoscope system having a capsule endoscope and a data reception device,
wherein the capsule endoscope includes:
an imaging module in which a frame rate of imaging is changeable based on a frame rate-setting value for designating the frame rate and configured to output imaging data after the imaging by performing the imaging; and
a first wireless communication interface configured to transmit the imaging data output from the imaging module to the data reception device and receive the frame rate-setting value from the data reception device, and
wherein the data reception device includes:
a second wireless communication interface configured to receive the imaging data from the capsule endoscope and transmit the frame rate-setting value to the capsule endoscope;
a storage module configured to store reference image information to be used to specify a part of a living body among the imaging data;
a range-of-interest detection unit configured to detect a range of interest which is a range of the part in the imaging data based on the reference image information;
an amount-of-change detection unit configured to detect an amount of change in the range of interest between the imaging data of two different frames; and
a frame rate determination unit configured to determine the frame rate-setting value to be transmitted to the capsule endoscope as a value greater than a previously determined frame rate-setting value when the amount of the change in the range of interest exceeds a predetermined reference value and determine the frame rate-setting value to be transmitted to the capsule endoscope as a value less than the previously determined frame rate-setting value when the amount of the change in the range of interest is less than the predetermined reference value.

10. A data reception method comprising the steps of:
receiving, by a wireless communication interface, imaging data transmitted from a capsule endoscope in which a frame rate of imaging is changeable based on a frame rate-setting value for designating the frame rate and which transmits imaging data after the imaging by performing the imaging;
detecting, by a range-of-interest detection unit, a range of interest which is a range of a part of a living body in the imaging data based on reference image information of a storage module configured to store the reference image information to be used to specify the part among the imaging data;
detecting, by an amount-of-change detection unit, an amount of change in the range of interest between the imaging data of two different frames;
determining, by a frame rate determination unit, the frame rate-setting value to be transmitted to the capsule endoscope as a value greater than a previously determined frame rate-setting value when the amount of the change in the range of interest exceeds a predetermined reference value and determining the frame rate-setting value to be transmitted to the capsule endoscope as a value less than the previously determined frame rate-setting value when the amount of the change in the range of interest is less than the predetermined reference value; and
transmitting, by the wireless communication interface, the determined frame rate-setting value to the capsule endoscope.

11. A program for causing a computer to execute the steps of:
causing a wireless communication interface to receive imaging data transmitted from a capsule endoscope in which a frame rate of imaging is changeable based on a frame rate-setting value for designating the frame rate and which transmits imaging data after the imaging by performing the imaging;
detecting a range of interest which is a range of a part of a living body in the imaging data based on reference image information of a storage module configured to store the reference image information to be used to specify the part among the imaging data;
detecting an amount of change in the range of interest between the imaging data of two different frames;
determining the frame rate-setting value to be transmitted to the capsule endoscope as a value greater than a previously determined frame rate-setting value when the amount of the change in the range of interest exceeds a predetermined reference value and determining the frame rate-setting value to be transmitted to the capsule endoscope as a value less than the previously determined frame rate-setting value when the amount of the change in the range of interest is less than the predetermined reference value; and
causing the wireless communication interface to transmit the determined frame rate-setting value to the capsule endoscope.
